(19) Europäisches Patentamt / European Patent Office / Office européen des brevets

(11) **EP 3 915 969 A1**

(12) **EUROPEAN PATENT APPLICATION**

(43) Date of publication:
01.12.2021 Bulletin 2021/48

(51) Int Cl.:
*C07C 51/235* (2006.01)    *B01J 37/08* (2006.01)

(21) Application number: 20386026.7

(22) Date of filing: 25.05.2020

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: ETH Zurich
8092 Zürich (CH)

(72) Inventors:
• **Mostrou-Moser, Sotiria**
**8048 Zürich (CH)**
• **VAN Bokhoven, Jeroen Anton**
**8044 Zürich (CH)**
• **Moser, Maximilian Karl-Rudolf Christian Werner**
**8048 Zürich (CH)**

(74) Representative: **Deblon, Jörg-Stephan**
**Patentanwaltskanzlei Dr. Deblon**
**Alte Honrather Strasse 22**
**53797 Lohmar (DE)**

(54) **A PROCESS FOR THE OXIDATION OF PRIMARY ALCOHOLS TO CARBOXYLIC ACIDS**

(57)    The present invention relates to a process for preparing carboxylic acids by oxidizing primary alcohols in the liquid phase in the presence of ruthenium dioxide as a catalyst.

Figure 1/1

**Description**

The present invention relates to a process for preparing carboxylic acids by oxidizing primary alcohols in the liquid phase in the presence of ruthenium dioxide ($RuO_2$) as a catalyst.

Carboxylic acids, in particular acetic acid, are key platform chemicals produced at very large industrial scale. The annual production of acetic acid for example exceeded 18 million metric tons in 2018 and was mainly consumed for the production of acetic anhydride, ester solvents such as ethyl acetate, propyl acetate, n-butyl and isobutyl acetate as well as for the production of vinyl acetate which itself is mainly used to manufacture polyvinyl acetate, a versatile polymer applied for example in glues, coatings or paints.

Currently, due to its abundance and low cost 75% of the global production of acetic acid is performed via the carbonylation of fossil methanol over homogeneous catalysts in the presence of hydrogen iodide additives.

Heterogeneously catalyzed oxidation of primary alcohols, such as ethanol, offers a highly selective and cost-effective route to convert such primary alcohols into the corresponding carboxylic acids. Utilization of bio-based primary alcohols such as bio-ethanol in such processes would allow a new route to introduce non-fossil feedstocks and chemicals into the global chemical value chain and thus decrease the eco-foot print of many large-scale industrial products.

However, the yields and selectivity of such heterogeneously catalyzed oxidations reported so far are not sufficient yet to compete with the currently used technologies.

In B. Jorgensen, S. Egholm Christiansen, M. L. Dahl Thomsen, C. H. Christensen, J. Catal. 2007, 251, 332-337 and S. Mostrou, T. Sipocz, A. Nagl, B. Fodi, F. Darvas, K. Föttinger, J. A. Van Bokhoven, React. Chem. Eng. 2018, 3, 781-789.; S. Mostrou, A. Nagl, M. Ranocchiari, K. Föttinger, J. A. van Bokhoven, Chem. Commun. 2019, 55, 11833-11836 and gold based catalysts supported on titanium dioxide are disclosed for the oxidation of ethanol to acetic acid. While showing high selectivity to acetaldehyde in the gas phase, see I. Sobolev, K. Y. Koltunov, O. A. Simakova, A.-R. Leino, D. Y. Murzin, Appl. Catal. A Gen. 2012, 433-434, 88-95), such catalytic systems suffer from various disadvantages when applied in the liquid phase for carboxylic acid production. Carbon dioxide is produced in larger amounts as a byproduct, and the catalytic system deactivates quite rapidly thereby increasing overall conversion costs to an unacceptable high level. Ruthenium based catalysts have shown to be more cost efficient and stable.

S. Muthusami et al., Tetrahedron Letters, 57, 2016, p. 5551 to 5559 summarizes "Recent advances in aerobic oxidation with ruthenium catalysts" inter alia for oxidation of primary and secondary alcohols.

A. B. Laursen, Y. Y. Gorbanev, F. Cavalca, P. Malacrida, A. Kleiman-Schwarstein, S. Kegnæs, A. Riisager, I. Chorkendorff, S. Dahl, Appl. Catal. A Gen. 2012, 433-434, 243-250 report about nanoparticulate mixed ruthenium oxide ($RuO_x$) catalysts on various supports, whereby cerium dioxide turned out to be the best among those tested. However, low selectivity and larger amounts of undesired byproducts in particular at higher temperatures are prohibitive for industrial use. Further, catalyst preparation is difficult and the final activity strongly depends on the preparation conditions, particle size and distribution on the selected support. Easy deactivation of the catalyst is reported with transformation of ruthenium (VI) to ruthenium (IV).

The same is true for mixed ruthenium hydroxide catalysts ($Ru(OH)_x$) inter alia on cerium oxide disclosed in Yury Y. Gorbanev, Søren Kegnæs, Christopher W. Hanning, Thomas W. Hansen, and Anders Riisager, ACS Catal. 2012, 2, 604-612. While a catalyst comprising 2.4 wt.-% of ruthenium hydroxides on cerium dioxide allows a conversion of ethanol of 92% and a yield of acetic acid of 78% in liquid phase oxidation after 3 hours at 150°C with a catalyst loading of 0.23 mol.-% (see table 2 therein, entry 8) the neat ruthenium hydroxide (table 2, entry 10) shows almost no activity under the same conditions.

In US 3,997,578, a ruthenium trichloride hydrate ($RuCl_3$ x $H_2O$) is employed to oxidize primary higher alcohols with four or more carbon atoms in the liquid phase. The oxidation, however, requires stoichiometric amounts of peracids as an oxidant. Ruthenium dioxide is listed as an alternative catalyst in claim 6 without, however, mentioning any details.

US 4,225,694 discloses oxidation of ethylenically unsaturated primary and secondary alcohols over alkali metal ruthenate in alkaline medium. The need to add Ruthenium dioxide ($RuO_2$) as well as alkali metal (per)halates and hypohalites throughout the reaction to reproduce the active alkali metal ruthenate renders the process unfavorable for larger scale applications.

H. Liu, E. Iglesia, J. Phys. Chem. B 2005, 109, 2155-2163 discloses oxidation of methanol and ethanol to formaldehyde and acetaldehyde respectively, over supported ruthenium dioxide (4.1 to 4.3 wt.-% Ruthenium) in the gas phase. However, in the gas phase there was no acetic acid formation.

Z. Opre, J.-D. Grunwaldt, M. Maciejewski, D. Ferri, T. Mallat, A. Baiker, J. Catal. 2005, 230, 406-419 report about Ruthenium-containing hydroxyapatite (RuHAp) catalysts for the oxidation of alcohols with molecular oxygen in an organic solvent. The activity of promoted RuHAp catalysts is inferior to those of supported platinum and palladium but they offer higher selectivity to the equivalent aldehydes. The study is focused on aromatic alcohols while carboxylic acids were not formed over RuHAp. In view of the prior art and the individual disadvantages associated with the chosen catalysts and process conditions there was still a need for a process with high space-time-yield using a highly selective, robust and durable catalyst.

A process has now been found for the preparation of carboxylic acids of formula (I)

$$R\text{-COOH} \qquad (I),$$

wherein R denotes alkyl, which is optionally further substituted once, twice or more than twice by aryl, hydroxy, halogen, cyano, alkoxy or aryloxy

The process comprising at least the step of reacting alcohols of formula (II) in the liquid phase

$$R\text{-CH}_2\text{-OH} \qquad (II),$$

wherein R has the meaning defined above

- with a gas comprising molecular oxygen, preferably dioxygen ($O_2$)

- in the presence of ruthenium dioxide ($RuO_2$).

The scope of the invention encompasses all combinations of substituent definitions, parameters and illustrations set forth above and below, either in general or within areas of preference or preferred embodiments, with one another, i.e., also any combinations between the particular areas and areas of preference.

Whenever used herein the terms "including", "e.g.", "such as" and "like" are meant in the sense of "including but without being limited to" or "for example without limitation", respectively.

As used herein the term "in the liquid phase" means that the alcohol of formula (II) whether used neat or in a diluent or solvent is kept liquid under chosen reaction conditions when contacted with ruthenium dioxide ($RuO_2$). It's apparent to those skilled in the art that this might require working under elevated pressure at higher temperatures.

As used herein, and unless specifically stated otherwise **alkyl** may be straight-chained, cyclic either in part or as a whole, branched or unbranched.

The term **$C_1$-$C_8$-alkyl** indicates that the straight-chained, cyclic either in part or as a whole, branched or unbranched alkyl substituent contains from 1 to 8 carbon atoms excluding the carbon atoms of optionally present substituents to the $C_1$-$C_8$-alkyl substituent. Specific examples of $C_1$-$C_8$-alkyl are methyl, ethyl, n-propyl, isopropyl, n-butyl, tert-butyl, n-pentyl, cyclohexyl, n-hexyl, n-heptyl, n-octyl, isooctyl.

As used herein, and unless specifically stated otherwise, **aryl** and **aryloxy** denotes carbocyclic aromatic or carbocyclic aryloxy substituents, whereby said carbocyclic, aromatic substituents or carbocyclic aryloxy substituents are unsubstituted or (further) substituted by up to three identical or different substituents per cycle. For example and with preference, the substituents are selected from the group consisting of fluorine, bromine, chlorine, nitro, cyano, $C_1$-$C_8$-alkyl, $C_1$-$C_8$-haloalkyl, $C_1$-$C_8$-alkoxy, $C_1$-$C_8$-haloalkoxy, $C_6$-$C_{14}$-aryl, in particular phenyl and naphthyl, di($C_1$-$C_8$-alkyl)amino, ($C_1$-$C_8$-alkyl)amino, $CO(C_1$-$C_8$-alkyl), $OCO(C_1$-$C_8$-alkyl), $NHCO(C_1$-$C_8$-alkyl), $N(C_1$-$C_8$-alkyl)$CO(C_1$-$C_8$-alkyl), $CO(C_6$-$C_{14}$-aryl), $OCO(C_6$-$C_{14}$-aryl), $NHCO(C_6$-$C_{14}$-aryl), $N(C_1$-$C_8$-alkyl)$CO(C_6$-$C_{14}$-aryl), $COO$-$(C_1$-$C_8$-alkyl), $COO$-$(C_6$-$C_{14}$-aryl), $CON(C_1$-$C_8$-alkyl)$_2$ or $CONH(C_1$-$C_8$-alkyl), $CONH_2$, $SO_2NH_2$, $SO_2N(C_1$-$C_8$-alkyl)$_2$, $SO_3M$ and $PO_3M_2$ with M being an alkali metal.

As used herein, and unless specifically stated otherwise **alkyl** may be straight-chained, cyclic either in part or as a whole, branched or unbranched. The same applies to **alkoxy.**

The term **$C_1$-$C_8$-alkyl** indicates that the straight-chained, cyclic either in part or as a whole, branched or unbranched alkyl substituent contains from 1 to 8 carbon atoms excluding the carbon atoms of optionally present substituents to the $C_1$-$C_8$-alkyl substituent. Specific examples of $C_1$-$C_8$-alkyl) are methyl, ethyl, n-propyl, isopropyl, n-butyl, tert-butyl, n-pentyl, cyclohexyl, n-hexyl, n-heptyl, n-octyl, isooctyl.

Specific examples of $C_1$-$C_4$-alkoxy-substituents are methoxy, ethoxy, isopropoxy, n-propoxy, n-butoxy and tert-butoxy. An additional example for $C_1$-$C_8$-alkoxy is cyclohexyloxy.

As used hereinabove, **$C_1$-$C_8$-haloalkyl** and **$C_1$-$C_8$-haloalkoxy** are **$C_1$-$C_8$-alkyl** substituents substituted by halogen atoms. Substituents which are fully substituted by fluorine are referred to as **$C_1$-$C_8$-perfluoroalkyl** and **$C_1$-$C_8$-perfluoroalkoxy,** respectively.

In a preferred embodiment R denotes **$C_1$-$C_8$-alkyl** which is either not or substituted once by alkoxy.

In a further preferred embodiment R is methyl, n-propyl or isopropyl meaning that preferred compounds of formula (I) are acetic acid, butyric acid and iso-butyric acid and are prepared from ethanol, n-butanol and iso-butanol respectively.

In one embodiment ethanol, n-butanol and iso-butanol as starting compounds of formula (II) are prepared from renewable sources e.g. from fermentation of monosaccharides by yeasts and bacteria.

A very preferred compound of formula (II) is ethanol which is converted to acetic acid.

To the extent liquid at the desired reaction temperature, the compounds of formula (II) may be applied in neat form or in a solvent that is not or virtually not prone to oxidation.

In particular, where water miscible compounds of formula (II) such as ethanol are employed they may be used as an aqueous solution at a level of from 0.5 to 95 vol.-%, preferably of from 1 to 90 vol.-%, more preferably of from 2 to 50 vol.-% and even more preferably of from 5 to 20 vol.-%.

Where compounds ethanol, n-butanol or iso-butanol are employed which are prepared from renewable sources e.g. from fermentation of monosaccharides by yeasts and bacteria, the crude filtrates from fermentation after removal of solid components may be employed as well. In case of ethanol such filtrates typically comprise of from 10 to 18 vol.-% of ethanol.

The compounds of formula (II) are reacted with a gas comprising molecular oxygen, preferably dioxygen ($O_2$). In one embodiment this includes pure dioxygen, mixtures of dioxygen and at least one inert gas such as nitrogen or a noble gas, or air each of them whether dried or not. In a very preferred embodiment air is employed as a gas comprising molecular oxygen.

The partial pressure of molecular oxygen, preferably dioxygen ($O_2$) is typically from 10 hPa to 10 MPa, preferably from 200 hPa to 1 MPa, more preferably from 0.1 MPa to 5 MPa and yet even more preferably from 0.5 MPa to 5 MPa. Higher pressures are possible but to the best of applicant's knowledge do not add any advantage.

The process according to the invention is carried out in the presence of ruthenium dioxide.

The ruthenium dioxide may be employed as amorphous or crystalline material, whereby crystalline material typically exhibits a rutile structure with most intense reflections at around 28.5° $2\theta$ and two further characteristic reflections at around 35.5° $2\theta$ and 54.5° $2\theta$ in powder X-ray diffraction.

Advantageously, ruthenium dioxide has a specific surface area of at least 1 $m^2/g$, preferably 1 to 300 $m^2/g$ and more preferably 2 to 200 $m^2/g$, and even more preferably 10 to 200 $m^2/g$ as measured by gas adsorption - BET method (ISO 9277:2010).

Advantageously, ruthenium dioxide has a crystal size as measured by powder X-ray diffraction according to the procedure given in the experimental part of 0.5 nm to 200 nm, preferably 1 nm to 50 nm and even more preferably 1 nm to 30 nm.

Where employed in flow reactors the ruthenium dioxide may be diluted i.e. physically mixed with or supported on materials such as silicon carbide (SiC), silica ($SiO_2$), alumina ($Al_2O_3$), titania ($TiO_2$), zirconium dioxide, zeolites, titanium composite oxides, zirconium composite oxides, aluminum composite oxides or other inert solid dilution or support materials.

Where ruthenium dioxide is used on support materials the content of ruthenium dioxide is preferably below 20 wt.-%, for example 1 to 20 wt.-% and preferably 1 to 5 wt.-%, in particular where higher mechanical stability of the catalytic material is required. In another embodiment a further catalytically active components can also be added, and examples of such further components include palladium compounds, copper compounds, chromium compounds, vanadium compounds, alkali metal compounds, rare earth compounds, manganese compounds and alkaline earth compounds. The amount of such further components is usually from 0.1 to 10 wt.-% based on the support material.

Ruthenium dioxide may, for example, be prepared by adding an alkali hydroxide to an aqueous solution of $RuCl_3$, thereby precipitating ruthenium hydroxide, washing the precipitate, followed by calcining in the air.

The support material can be used, for example, in the form of powder with particle sizes of 0.001 to 0.1 mm, crushed and sieved material with particle sizes between 0.05 and 5 mm.

In this case the amount of ruthenium dioxide in the catalyst material may be for example 0.1 to 35 wt.-%, preferably 1 to 10 wt.-%.

The use of ruthenium dioxide in the processes as described herein was not reported before. Therefore, the invention also encompasses the use of ruthenium dioxide for the manufacture of compounds of formula (I) via oxidation of compounds of formula (II) with a gas comprising molecular oxygen, preferably dioxygen ($O_2$) in the liquid phase.

The process according to the invention can be performed batchwise or continuously, preference being given to continuous performance.

In batchwise processes, the weight ratio of compounds of formula (II) to ruthenium dioxide is typically from 250 to 4000, preferably from 500 to 1500. The reaction times are typically from 15 minutes to 24 hours, preferably from 1 hour to 12 hours.

In continuous processes, the throughput is typically selected such that the weight ratio of compounds of formula (II) to ruthenium dioxide is typically from 50 to 4000, preferably from 100 to 1500 per hour.

The residence times are for example from 1 minute to 3 hours, preferably from 3 minutes to 60 minutes.

The service life of ruthenium dioxide in continuous processes ranges from 1 hour to 2000 hours or more. A significant degradation or inactivation could not be observed.

Since the reaction proceeds via two steps, first the oxidation to the corresponding aldehyde and then to the desired carboxylic acids of formula (I); higher selectivity to the carboxylic acids of formula (I) require some more time as can be seen in the experimental part and are strongly dependent on temperature.

In one embodiment the reaction is performed such that at least 20 % of the mass of compound of formula (II) employed is converted, preferably 20 to 100 %, more preferably 30 to 80 %, even more preferably 30 to 60 % and yet even more preferably35 to 50 %.

In one embodiment the reaction is performed such that 0.5 to 20.0, preferably 0.5 to 5.0 and more preferably 0.5 to 3.0 g of acetic acid are produced per g of Ruthenium dioxide and hour. The process according to the invention can be performed, for example, in any reactor allowing a triphasic reaction known to those skilled in the art, i.e. a three-phase fixed bed reactor, a trickle flow reactor e.g. a trickle film or trickle bed reactor, a fluidized bed reactor or a suspension reactor for example a bubble column reactor or a stirred tank with gas inlet. In a preferred embodiment the reaction is performed in a trickle flow reactor.

The process according to the invention is performed, for example, at a reaction temperature of 75 to 250°C, preferably 100 to 220°C, more preferably 130 to 220°C and even more preferably 140 to 200°C.

The advantage of the present invention is that the process according to the invention using ruthenium dioxide as a catalyst in the liquid phase allows to obtain the desired carboxylic acids of formula (I) with high selectivity, high weight hourly space velocities and thus good space time yields and exhibits high robustness even after prolonged reaction times.

The following examples are intended to illustrate the invention, but without limiting it thereto.

**Experimental part** - **Examples**

General

A) $RuO_2$ catalysts

The $RuO_2$ catalysts employed herein were commercially obtained from

1) Acros Organics, with a purity of 99.5+%, a crystal size $t$ of 27.0 nm as measured by X-ray diffraction and a specific surface area $S_{BET}$ of 17 m$^2$/g (hereinafter referred to as $RuO_2$ - Type 1)

2) Alfa Aesar with a purity of 99.95%, a crystal size $t$ of 1.5 nm as estimated by X-ray diffraction and a specific surface area $S_{BET}$ of 107 m$^2$/g (hereinafter referred to as $RuO_2$ - Type 2)

or derived from the aforementioned by

3) Calcinating $RuO_2$ - Type 2 at a temperature of 823 K for 5 hours, thereby producing a ruthenium dioxide having a crystal size $t$ of 31.3 nm as measured by X-ray diffraction and a specific surface area $S_{BET}$ of 2.8 m$^2$/g (hereinafter referred to as $RuO_2$ - Type 3)

Notes:

The specific surface areas were determined by gas adsorption - BET method (ISO 9277:2010).

The crystal size $t$ was evaluated by the the reflection at about 54.5° $2\theta$ of the X-ray diffraction after fitting the Bragg reflections to Gaussian and Lorentzian functions using the software TOPAS 6 [A. A. Coelho, J. Appl. Crystallogr. 2018, 51, 210-218]. The full width at half maximum was deconvoluted from these fittings and the crystal size was calculated by using Scherrer equation according to the method disclosed in P. Scherrer, Kolloidchem. Ein Lehrbuch (Ed.: R. Zsigmondy), Springer Berlin Heidelberg, Berlin, Heidelberg, 1912, pp. 387-409. The X-ray diffraction patterns were acquired on a PANalytical X'Pert PRO-MPD diffractometer, operating with Cu(K$\alpha$) radiation. Data were recorded in the 10-70° $2\theta$ range with an angular step size of 0.050° and a counting time of 2 s per step.

B) The reactor

The experiments were performed in a custom-made trickle-flow reactor.

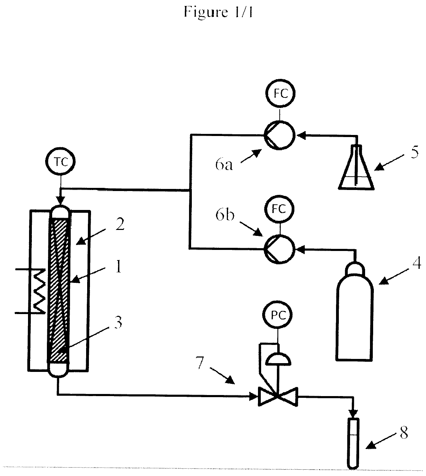

Figure 1 shows a simplified depiction thereof. The numerals indicate the following:

1    reactor
2    metal plate heater
3    catalyst bed
4    oxygen bottle
5    ethanol supply
6a   high-pressure liquid chromatography pump
6b   mass flow controller

7 back-pressure regulator

8 product collection

The reactor was operated as follows:
Oxygen (PanGas, 99.999%) was supplied from an oxygen bottle 4 via a mass flow controller **6b** by Bronkhorst, calibrated for oxygen flow at 20 bar (4). The liquid flow (5 $\pm$ 0.3 wt.-% ethanol solution (Fluka, >99.8%)) was introduced from the ethanol reservoir **5** with a KNAUER AZURA® P 4.1S high-pressure liquid chromatography pump, equipped with a titanium 10 ml pump head **6a.** The two phases were met and introduced to the reactor **1** via Swagelok 1/8" stainless steel tubing. Before entering the reactor **1,** the reactant stream was preheated to about 120°C. The catalyst bed **3** (150 $\pm$ 0.1 mg catalyst diluted 1:1 with SiC) was fixed with quartz wool inside a 4 mm inner diameter stainless steel tube (reactor); The bed was stabilized in the middle of the heating zone by a hollow stainless-steel rod of ca. 1.5 outer diameter, which ensured a constant height and minimum back pressure (maximum 0.2 bar). A custom-made metal plate heater **2** heated the reactor; it was controlled by a temperature controller (TC). An Equilibar® U3L Series precision back-pressure regulator **7,** equipped with a PTFE glass laminated diaphragm, maintained the pressure of the system. The back-pressure regulator 7 was controlled by a Bronkhorst process pressure controller EL-PRESS P-802CV (PC). The flows, temperature of the heater, and the pressure of the back-pressure regulator were all recorded and controlled via a custom-made Lab-VIEW™program. The temperature of the catalyst bed was recorded with a K-type thermocouple. The system pressure was also recorded before the reactor with a Keller Digital Manometer dV-2 PS. The product stream was collected in the product collection **8** and cooled down below 280 K by a dry ice-water bath and prepared for sampling.

If not indicated otherwise the process conditions were as follows:

| | |
|---|---|
| Reaction Pressure | 17 $\pm$ 1.0 |
| Temperature (°C) | 150 $\pm$ 3 |
| Ruthenium dioxide mass (mg) | 150 $\pm$ 2 |
| Ruthenium dioxide dilution ratio with SiC | 1:1 |
| Ethanol concentration (wt-%) | 5 $\pm$ 0.3 |
| Oxygen purity (vol-%) | 100 |
| Liquid flow (mL/min) | 0.3 |
| Gas flow (mL/min) | 5 |
| Residence time (min) | 5.5 |
| Product cooling (K) | 288 $\pm$ 2 |

C) Product Analyses

The liquid products were analyzed with an Agilent 7890A gas chromatographer equipped with a flame ionization detector (FID). 0.5 $\mu$L of the sample were injected at 343 K and carried in a 2 mL/min helium flow through the column DB-WAX. The temperature of the column was constant at 313 K for 2 min and was then heated at 8 K/min up to 409 K. The FID was fed by 30 mL/min hydrogen mixed in 400 mL/min air at 573 K. The signal of each compound was calibrated and the calibration line used for quantification was determined by linear regression.

The quantification of the compounds was used to determine the ethanol conversion ($X$) and the product selectivity ($S_i$), wherein EtOH is ethanol and AcOH is acetic acid.

$$X(\%) = EtOHin\ (EtOHmol/l)_{in}\text{-}EtOH(mol/lout)\ (mol/l)\ \text{x}\ 100\%$$

$$SAcOH\ (\%) = EtOHin\ (AcOHmol/lout)\text{-}EtOH(mol/lout)\ (mol/l)\ \text{x}\ 100\%$$

Examples 1 to 8

Examples 1 to 8 were run using the above mentioned setup at different temperatures using $RuO_2$ - Type 1 diluted with SiC (1:1) as a catalyst.

The results are given in table 1

Table 1

| Example | Temperature [C] | Conversion ($X$) | Y_AcOH | S_AcOH |
|---------|-----------------|------------------|--------|--------|
| 1 | 100 | 6.8 | 3.9 | 56.6 |
| 2 | 100 | 9.3 | 5.6 | 60.0 |
| 3 | 125 | 18.3 | 13.3 | 72.6 |
| 4 | 150 | 21.5 | 18.3 | 85.0 |
| 5 | 150 | 22.9 | 17.7 | 77.2 |
| 6 | 150 | 25.1 | 20.1 | 80.2 |
| 7 | 150 | 27.5 | 25.4 | 92.3 |
| 8 | 170 | 37.9 | 37.7 | 99.3 |

It's apparent from the results that the selectivity to acetic acid is almost quantitative at conversions about and above 35 % of ethanol which is achieved in this experimental setup at temperatures of 170 °C.

Examples 9 to 11

Examples 9 to 1 I were run using the above mentioned setup with various types of ruthenium dioxide diluted with SiC (1:1) at 150°C
The results are given in table 2.

Table 2:

| Example | Catalyst | Conversion [X) | Y_AcOH | S_AcOH | g AcOH g $RuO_2$ x h |
|---------|----------|----------------|--------|--------|----------------------|
| 9 | $RuO_2$ - Type 1 | 25.4 | 17.3 | 68.1 | 1.2 |
| 10 | $RuO_2$ - Type 2 | 48.4 | 39.5 | 81.6 | 2.4 |
| 11 | $RuO_2$ - Type 3 | 17.2 | 11.0 | 64.1 | 0.6 |

Example 12

Example 12 was performed using the above mentioned setup with ruthenium dioxide - type 1 diluted with SiC (1:1) at 150°C for more than 24 hours.
The results are given in table 3:

Table 3:

| Time [h] | Conversion [X] | Y_(AcOH) | S (AcOH) |
|----------|----------------|----------|----------|
| 7 | 25.6 | 18.4 | 72.0 |
| 11 | 21.5 | 18.3 | 85.0 |
| 19 | 22.9 | 17.7 | 77.2 |
| 27 | 20.9 | 18.7 | 89.6 |

It is apparent from table 3 that the catalyst is stable over time and does virtually not deactivate.

**Claims**

1.   A process for the preparation of carboxylic acids of formula (I)

R-COOH            (I),

wherein R denotes alkyl, which is optionally further substituted once, twice or more than twice by aryl, hydroxy, halogen, cyano, alkoxy or aryloxy
the process comprising at least the step of reacting alcohols of formula (II) in the liquid phase

$$R\text{-}CH_2\text{-}OH \qquad (II),$$

wherein R has the meaning defined above

- with a gas comprising molecular oxygen, preferably dioxygen ($O_2$)
- in the presence of ruthenium dioxide ($RuO_2$).

2.  The process according to claim 1, wherein in formulae (I) and (II) R is $C_1$-$C_8$-alkyl which is either not or substituted once by alkoxy.

3.  The process according to claim 1 or 2, wherein in formulae (I) and (II) R is methyl, n-propyl or isopropyl, preferably methyl.

4.  The process according to claims 3, wherein the compounds of formula (II) employed are prepared from renewable resources, preferably by fermentation of monosaccharides by yeasts and bacteria.

5.  The process according to any one of claims 1 to 4, wherein the compounds of formula (II) are water miscible and are employed as an aqueous solution at a level of from 0.5 to 95 vol.-%, preferably of from 1 to 90 vol.-%, more preferably of from 2 to 50 vol.-% and even more preferably of from 5 to 20 vol.-%.

6.  The process according to any one of claims 1 to 5, wherein the gas comprising molecular oxygen, preferably dioxygen ($O_2$) is selected from pure dioxygen, mixtures of dioxygen and at least one inert gas, or air each of them whether dried or not.

7.  The process according to any one of claims 1 to 6, wherein the partial pressure of molecular oxygen, preferably dioxygen ($O_2$) is typically from 10 hPa to 10 MPa, preferably from 200 hPa to 1 MPa, more preferably from 0.1 MPa to 5 MPa and yet even more preferably from 0.5 MPa to 5 MPa.

8.  The process according to any one of claims 1 to 7, wherein the ruthenium dioxide has specific surface area of at least 1 $m^2$/g, preferably 1 to 300 $m^2$/g and more preferably 2 to 200 $m^2$/g, and even more preferably 10 to 200 $m^2$/g as measured by gas adsorption according to BET method (ISO 9277:2010).

9.  The process according to any one of claims 1 to 8, wherein the ruthenium dioxide has crystal size as measured by powder X-ray diffraction of 0.5 nm to 200 nm, preferably 1 nm to 50 nm and even more preferably 1 nm to 30 nm.

10. The process according to any one of claims 1 to 9, wherein the ruthenium dioxide is diluted with or supported on inert solid materials.

11. The process according to any one of claims 1 to 10 being performed batchwise or continuously, preferably continuously.

12. The process according to any one of claims 1 to 11 being performed continuously with residence times of 1 minute to three hours.

13. The process according to any one of claims 1 to 12 being performed such that at least 20 % of the mass of compound of formula (II) employed is converted, preferably 20 to 100 %, more preferably 30 to 80 %, even more preferably 30 to 60 % and yet even more preferably 35 to 50 %.

14. The process according to any one of claims 1 to 13, wherein a three-phase fixed bed reactor, a trickle flow reactor, a fluidized bed reactor or a suspension reactor a stirred tank with gas inlet is employed to host the reaction.

15. The process according to any one of claims 1 to 14, wherein the reaction temperature is 75 to 250°C, preferably 100 to 220°C, more preferably 130 to 220°C and even more preferably 140 to 200°C.

Figure 1/1

Europäisches
Patentamt

European
Patent Office

Office européen
des brevets

# EUROPEAN SEARCH REPORT

Application Number

EP 20 38 6026

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | OPRE Z ET AL: "Promoted Ru-hydroxyapatite: designed structure for the fast and highly selective oxidation of alcohols with oxygen", JOURNAL OF CATALYSIS, ACADEMIC PRESS, DULUTH, MN, US, vol. 230, no. 2, 10 March 2005 (2005-03-10), pages 406-419, XP004762408, ISSN: 0021-9517, DOI: 10.1016/J.JCAT.2004.12.012 * the whole document * * figures 3,4 * * table 1 * | 1-15 | INV. C07C51/235 B01J37/08 |
| A | WO 2017/208098 A1 (SABIC GLOBAL TECHNOLOGIES BV [NL]) 7 December 2017 (2017-12-07) * claim 1 * | 1-15 | |

TECHNICAL FIELDS SEARCHED (IPC)

C07C
B01J

The present search report has been drawn up for all claims

| Place of search | Date of completion of the search | Examiner |
|---|---|---|
| The Hague | 12 November 2020 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 38 6026

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2020

| Patent document cited in search report | Publication date | Patent family member(s) | Publication date |
|---|---|---|---|
| WO 2017208098 A1 | 07-12-2017 | CN 109195937 A<br>DE 112017002703 T5<br>US 2019315672 A1<br>WO 2017208098 A1 | 11-01-2019<br>14-02-2019<br>17-10-2019<br>07-12-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3997578 A
- US 4225694 A

**Non-patent literature cited in the description**

- **B. JORGENSEN ; S. EGHOLM CHRISTIANSEN ; M. L. DAHL THOMSEN ; C. H. CHRISTENSEN.** *J. Catal.,* 2007, vol. 251, 332-337
- **S. MOSTROU ; T. SIPŐCZ ; A. NAGL ; B. FŐDI ; F. DARVAS ; K. FÖTTINGER ; J. A. VAN BOKHOVEN.** *React. Chem. Eng.,* 2018, vol. 3, 781-789
- **S. MOSTROU ; A. NAGL ; M. RANOCCHIARI ; K. FÖTTINGER ; J. A. VAN BOKHOVEN.** *Chem. Commun.,* 2019, vol. 55, 11833-11836
- **I. SOBOLEV ; K. Y. KOLTUNOV ; O. A. SIMAKOVA ; A.-R. LEINO ; D. Y. MURZIN.** *Appl. Catal. A Gen.,* 2012, vol. 433-434, 88-95
- **S. MUTHUSAMI et al.** Recent advances in aerobic oxidation with ruthenium catalysts. *Tetrahedron Letters,* 2016, vol. 57, 5551-5559
- **A. B. LAURSEN ; Y. Y. GORBANEV ; F. CAVALCA ; P. MALACRIDA ; A. KLEIMAN-SCHWARSTEIN ; S. KEGNÆS ; A. RIISAGER ; I. CHORKENDORFF ; S. DAHL.** *Appl. Catal. A Gen.,* 2012, vol. 433-434, 243-250
- **YURY Y. GORBANEV ; SØREN KEGNÆS ; CHRISTOPHER W. HANNING ; THOMAS W. HANSEN ; ANDERS RIISAGER.** *ACS Catal.,* 2012, vol. 2, 604-612
- **H. LIU ; E. IGLESIA.** *J. Phys. Chem. B,* 2005, vol. 109, 2155-2163
- **Z. OPRE ; J.-D. GRUNWALDT ; M. MACIEJEWSKI ; D. FERRI ; T. MALLAT ; A. BAIKER.** *J. Catal.,* 2005, vol. 230, 406-419
- **A. A. COELHO.** *J. Appl. Crystallogr.,* 2018, vol. 51, 210-218
- **P. SCHERRER.** Kolloidchem. Ein Lehrbuch. Springer, 1912, 387-409